# EUROPÄISCHE PATENTSCHRIFT

(11) **EP 3 413 053 B1**
(45) Veröffentlichungstag und Bekanntmachung des Hinweises auf die Patenterteilung: **06.01.2021**
(21) Anmeldenummer: 17174927.8
(22) Anmeldetag: 08.06.2017
(51) Int. Cl.: G01N 33/86

(54) **KALIBRATION VON LUPUS ANTIKOAGULANS**
CALIBRATION OF LUPUS ANTICOAGULANT
ÉTALONNAGE D'ANTICOAGULANT LUPIQUE

(43) Veröffentlichungstag der Anmeldung: 12.12.2018
(73) Patentinhaber: Siemens Healthcare Diagnostics Products GmbH, 35041 Marburg (DE)
(72) Erfinder: Patzke, Juergen, 35043 Marburg (DE)

(56) Entgegenhaltungen:
- EP-A1- 0 841 566
- EP-A2- 0 898 168
- WO-A1-00/02925
- WO-A1-2007/018511
- US-A- 4 877 741
- SCHJETLEIN R ET AL: "A quantitative, semi-automated and computer-assisted test for lupus anticoagulant", THROMBOSIS RESEARCH, TARRYTOWN, NY, US, Bd. 69, Nr. 2, 15. Januar 1993 (1993-01-15), Seiten 239-250, XP026465300, ISSN: 0049-3848, DOI: 10.1016/0049-3848(93)90049-T [gefunden am 1993-01-15]
- QUERREC LE A ET AL: "QUANTIFICATION OF LUPUS ANTICOAGULANTS IN CLINICAL SAMPLES USING ANTI-BETA2GP1 AND ANTI-PROTHROMBIN MONOCLONAL ANTIBODIES", THROMBOSIS AND HAEMOSTASIS, SCHATTAUER GMBH, DE, Bd. 86, Nr. 2, 1. August 2001 (2001-08-01) , Seiten 584-589, XP001080224, ISSN: 0340-6245

## Beschreibung

Die Erfindung liegt auf dem Gebiet der Gerinnungsdiagnostik und betrifft ein Kalibratormaterial zur Kalibration von Gerinnungstesten, die dem Nachweis von Lupus antikoagulans dienen.

Lupus antikoagulans sind Immunglobuline und gehören zum Formenkreis der erworbenen Autoantikörper. Sie verursachen das Antiphospholipid-Syndrom (APS), eine der häufigsten Autoimmunerkrankungen, und rufen Thrombosen, wiederkehrende Fehlgeburten sowie Schwangerschaftskomplikationen hervor. Die Lupus antikoagulans Immunoglobuline sind sogenannte Antiphospholipid-Antikörper (APA), die an anionische Phospholipide, an Proteine oder an Protein/ Phospholipid-Komplexe binden. Die Antiphospholipid-Antikörper, die mit bestimmten Proteinen und Phospholipiden Komplexe bilden, stellen eine sehr heterogene Gruppe von Autoantikörpern dar, die gegen eine Vielzahl von Antigenen gerichtet sein können, wie z.B. gegen das Apolipoprotein β2-Glykoprotein I (β2GPI), Cardiolipin, Prothrombin, Protein C, Protein S, Thrombomodulin, Faktor XII und andere, sowie gegen Komplexe dieser Proteine mit Phospholipiden.

Die sogenannten Lupus Antikoagulanzien sind Antiphospholipid-Antikörper, die definitionsgemäß die Gerinnungszeiten von bestimmten Gerinnungstesten, z.B. von der APTT verlängern. Paradox ist, dass die Lupus Antikoagulanzien in vitro eine Hemmung der Gerinnungsreaktion bewirken, während in vivo eine erhöhte Gerinnungsreaktion (Hyperkoagulabilität) mit dem Antiphospholipid-Syndrom (APS) einhergeht.

Die Labordiagnose eines Antiphospholipid-Syndroms (APS) wird durch die Heterogenität der Antiphospholipid-Antikörper erschwert. Zum direkten Nachweis der Antikörper werden immunologische Verfahren angewandt. Hierbei werden allerdings auch viele Antikörper erfasst, die in vivo nicht prothrombotisch wirksam sind. Zum indirekten Nachweis der Antikörper werden Gerinnungsteste angewandt. Bei einem Gerinnungstest wird eine Plasmaprobe des Patienten typischerweise mit einem Gerinnungsaktivator, Phospholipiden und Calciumionen vermischt, und es wird die Zeit bis zur Gerinnselbildung gemessen. Besonders Teste, die auf der Bestimmung der DRVVT (Dilute Russell's Viper Venom Time) basieren, zeigen eine relativ gute Korrelation zur prothrombotischen Wirksamkeit der Antiphospholipid-Antikörper. Die Lupus-Diagnostik ist sehr aufwändig, weil jede Patientenprobe mehrere Untersuchungsschritte durchlaufen muss (zur Übersicht: Devreese, K. and Hoylaerts, M.F., Challenges in the diagnosis of the antiphospholipid syndrome. Clin. Chem. 2010, 56(6): 930-940).

Typischerweise wird eine Patientenprobe zur Diagnose eines Lupus antikoagulans mit zwei Varianten mindestens eines Gerinnungstestes untersucht. In der ersten, Lupus antikoagulans-sensitiven Variante wird der Test in Gegenwart einer relativ geringen Phospholipid-Konzentration durchgeführt; in der zweiten, Lupus antikoagulans-insensitiven Variante wird derselbe Test in Gegenwart einer relativ hohen Phospholipid-Konzentration durchgeführt. Eine gegenüber normalen Proben (die kein Lupus antikoagulans enthalten) verlängerte Gerinnungszeit in der ersten Testvariante in Kombination mit einer gegenüber normalen Proben normalen Gerinnungszeit in der zweiten Testvariante spricht für das Vorliegen von Lupus antikoagulans.

Problematisch ist, dass jeder Gerinnungstest für sich allein nicht spezifisch ist für Lupus antikoagulans. Die Teste reagieren auch in unterschiedlichem Ausmaß auf Gerinnungsfaktormängel oder therapeutische Antikoagulanzien in einer Probe.

Ein besonders häufig eingesetzter Test ist der DRVVT-Test, wobei die erste, Lupus antikoagulans-sensitive Variante mit einem Aktivierungsreagenz mit Russel-Viper-Gift und einer relativ geringen Phospholipid-Konzentration (LA1 Screeningreagenz) durchgeführt wird und die zweite, Lupus antikoagulans-insensitive Variante mit einem Aktivierungsreagenz mit Russel-Viper-Gift und einer hohen Phospholipid-Konzentration (LA2 Bestätigungsreagenz) durchgeführt wird. Ist der LA1-Test negativ, d.h. wird keine gegenüber dem Normal verlängerte Gerinnungszeit gemessen, ist die Durchführung des LA2-Testes nicht mehr notwendig.

Die Bildung des LA1/LA2-Ratios (Quotienten) aus den beiden Testergebnissen spiegelt die Phospholipid-Abhängigkeit der Verlängerung der LA1-Gerinnungszeit wieder und ist damit relativ spezifisch für Lupus antikoagulans. Starke Gerinnungsfaktormängel oder hohe Konzentrationen therapeutischer Antikoagulanzien in einer Probe können jedoch auch das Ratio beeinflussen, so dass kein spezifischer Nachweis eines Lupus antikoagulans möglich ist.

Sowohl der LA1-Test als auch der LA2-Test und damit auch das LA1/LA2-Ratio können von Labor zu Labor, von Analysegerät zu Analysegerät, von Reagenzcharge zu Reagenzcharge für ein und dieselbe Probe zum Teil stark abweichende Testergebnisse liefern. Diese Varianz der Testergebnisse, die im Übrigen nicht nur für DRVVT-basierte Teste, sondern auch für Teste, die auf der Bestimmung anderer Gerinnungszeiten basieren, zutrifft, erschwert die Festlegung eines medizinischen Entscheidungspunktes, ab dem ein Ergebnis als pathologisch einzustufen ist. Daher ist die Kalibration von Gerinnungstest-basierten Lupus antikoagulans-Testen wünschenswert.

Da ein Lupus-sensitiver Gerinnungstest das Vorliegen von Lupus antikoagulans detektieren soll, sollte ein geeignetes Kalibratormaterial typischerweise Lupus antikoagulans enthalten. Le Querrec et al. schlagen dazu einen Normalplasmapool vor, dem zwei unterschiedliche Lupus antikoagulans Immunoglobuline, ein anti-β2-Glykoprotein I-Antikörper und ein anti-Prothrombin-Antikörper, zugegeben sind (Le Querrec, A. et al., Quantification of lupus anticoagulants in clinical samples using anti-β2-GP1 and anti-prothrombin monoclonal antibodies. Thromb Haemost 2001, 86: 584-589). In der EP-A2-0898168 wird zur Simulation von Lupus antikoagulans ein Annexin einer Plasmamatrix zugesetzt.

Allerdings zeigen auch die Lupus antikoagulans-insensitiven Gerinnungsteste von den unterschiedlichen Einflussfaktoren beeinflusste, variierende Testergebnisse, so dass auch hierfür eine Kalibration wünschenswert wäre. Idealerweise sollte ein einziges Kalibrationsmaterial sowohl für den Lupus antikoagulans-sensitiven als auch den Lupus antikoagulans-insensitiven Test verfügbar sein, um die Komplexität bei der Abarbeitung möglichst gering zu halten.

Der vorliegenden Erfindung lag also die Aufgabe zugrunde, ein Kalibratormaterial bereit zu stellen, das sich für die Kalibration von Gerinnungstesten, insbesondere von Lupus antikoagulans-sensitiven und den dazugehörigen Lupus antikoagulans-insensitiven Gerinnungstesten, eignet, die für die Bestimmung von Lupus antikoagulans durchgeführt werden.

Die Aufgabe wird dadurch gelöst, dass ein Kalibratormaterial bereitgestellt wird, das aus Plasma besteht, welches im Vergleich zu humanem Normalplasma einen Mangel an mindestens einem Gerinnungsfaktor aus der Gruppe Faktor II und Faktor X aufweist, wobei das Plasma im Vergleich zu Normalplasma 0 % Faktor II und/oder 0 % Faktor X enthält und welches ferner mindestens eine Lupus antikoagulans-auslösende Substanz enthält.

Es wurde festgestellt, dass die Kalibration von Testergebnissen von Lupus antikoagulans-sensitiven und den dazugehörigen Lupus antikoagulans-insensitiven Gerinnungstesten mit einer Kalibrationskurve, die mit erfindungsgemäßen Kalibratormaterialien erstellt wurde, Reagenzchargen- sowie Analysegerät-bedingte Testergebnisabweichungen vermindert und somit eine bessere Vergleichbarkeit der Testergebnisse bewirkt.

Gegenstand der vorliegenden Erfindung ist also ein Kalibratormaterial wie in Anspruch 1 beschrieben.

Ein Plasma, das einen Mangel an mindestens einem Gerinnungsfaktor aufweist, weist immer eine geringere Gerinnungsfaktorkonzentration auf als ein humanes Normalplasma, dessen Gerinnungsfaktorkonzentration per definitionem 100 % beträgt. Bei dem Plasma, das einen Mangel an mindestens einem Gerinnungsfaktor aufweist, handelt es sich vorzugsweise um humanes Plasma, besonders bevorzugt um Normalplasma eines gesunden Spenders oder um einen Normalplasmapool aus Normalplasmen mehrerer gesunder Spender, die keine offensichtliche Gerinnungsstörung aufweisen, dem der eine oder mehrere Gerinnungsfaktoren, wie z.B. Faktor II (Prothrombin) und/oder Faktor X, vollständig entzogen wurden. Bekannte Verfahren zur Herstellung von Gerinnungsfaktor-Mangelplasma sind beispielsweise immunaffinitätschromatographische Methoden. Alternativ kann es sich bei dem Plasma, das einen Mangel an mindestens einem Gerinnungsfaktor aufweist, auch um ein tierisches Plasma, wie z.B. Rinderplasma oder Kaninchenplasma, oder um eine Mischung von humanem und tierischem Plasma handeln.

Erfindungsgemäß weist das Plasma, aus dem das Kalibratormaterial besteht, einen Mangel an Faktor II und/oder Faktor X auf. Je höher der Faktormangel desto größer ist die Gerinnungszeitverlängerung in Lupus antikoagulans-sensitiven und den dazugehörigen Lupus antikoagulans-insensitiven Gerinnungstesten.

Erfindungsgemäß enthält das Plasma, aus dem das Kalibratormaterial besteht, im Vergleich zu humanem Normalplasma 0 % Faktor II und/oder 0 % Faktor X.

Dieses Mangelplasma, aus dem das Kalibratormaterial besteht, enthält ferner eine Lupus antikoagulans-auslösende Substanz. Unter einer "Lupus antikoagulans-auslösenden Substanz" sind einerseits Substanzen zu verstehen, die in vivo ein Antiphospholipid-Syndrom verursachen, d.h. Antiphospholipid-Antikörper, und andererseits Substanzen, die physiologisch nicht ursächlich für ein Antiphospholipid-Syndrom sind, sondern lediglich in vitro ein Lupus antikoagulans simulieren, weil sie in Bezug auf die in vitro-Diagnostik dieselben funktionellen Eigenschaften aufweisen wie ein Lupus antikogulans, wie beispielsweise Annexine. In EP-A2-0898168, Absatz 0008 sind die funktionellen Kriterien für in vitro Lupus antikoagulans-auslösende Substanzen beschrieben.

Ein bevorzugtes Kalibratormaterial besteht aus Plasma, das als Lupus antikoagulans-auslösende Substanz mindestens einen Antiphospholipid-Antikörper enthält. Vorzugsweise kann das Plasma zwei, drei, vier oder mehr unterschiedliche Antiphospholipid-Antikörper enthalten. Bevorzugterweise enthält das Plasma einen, zwei oder mehr Antiphospholipid-Antikörper aus der Gruppe anti-β2-Glykoprotein I-Antikörper, anti-Prothrombin-Antikörper, anti-Cardiolipin-Antikörper, anti-Protein C-Antikörper, anti-Protein S-Antikörper, anti-Thrombomodulin-Antikörper und anti-Faktor XII-Antikörper.

Bevorzugterweise ist der jeweilige Antikörper in einer Konzentration von 0,5 bis 500 µg/mL in dem Plasma, aus dem das Kalibratormaterial besteht, enthalten.

Ein anderes bevorzugtes Kalibratormaterial besteht aus Plasma, das als Lupus antikoagulans-auslösende Substanz mindestens ein Annexin, vorzugsweise Annexin V enthält. Bevorzugterweise ist das jeweilige Annexin in einer Konzentration von 0,5 bis 500 µg/mL in dem Plasma, aus dem das Kalibratormaterial besteht, enthalten.

Ein besonders bevorzugtes erfindungsgemäßes Kalibratormaterial besteht aus Plasma, dem mindestens ein Gerinnnungsfaktor vollständig entzogen wurde (z.B. 0 % Faktor II und/oder 0 % Faktor X) und das mindestens einen Antiphospholipid-Antikörper in einer Konzentration von 50 bis 500 µg/mL enthält. Dieses Kalibratormaterial eignet sich als "Stammkalibratormaterial", aus dem durch Zugabe von humanem Normalplasma Kalibratormaterialien von sukzessive zunehmender Gerinnnungsfaktorkonzentration und gleichzeitig sukzessive abnehmender Antikörperkonzentration herstellen lassen.

Ein weiterer Gegenstand der vorliegenden Erfindung ist ein Verfahren zur Herstellung eines erfindungsgemäßen Kalibratormaterials, bei welchem dem Plasma, das im Vergleich zu humanem Normalplasma 0 % Faktor II und/oder 0 % Faktor X enthält, eine definierte Menge mindestens einer Lupus antikoagulans-auslösenden Substanz zugegeben wird. Noch ein weiterer Gegenstand der vorliegenden Erfindung ist ein Kalibratorset enthaltend ein erstes Gefäß enthaltend ein erfindungsgemäßes Kalibratormaterial und optional ein zweites Gefäß enthaltend humanes Normalplasma. Bei dem in dem ersten Gefäß enthaltenen Kalibratormaterial handelt es sich vorzugsweise um das weiter oben beschriebene "Stammkalibratormaterial", aus dem sich durch Zugabe von dem in dem zweiten Gefäß enthaltenen humanem Normalplasma Kalibratormaterialien von sukzessive zunehmender Gerinnnungsfaktorkonzentration und gleichzeitig sukzessive abnehmender Antikörperkonzentration herstellen lassen.

Alternativ kann ein erfindungsgemäßes Kalibratorset auch zwei, drei, vier oder mehr Gefäße enthalten, wobei in jedem Gefäß ein Kalibratormaterial mit einem definierten Gerinnungsfaktormangel und einer definierten Konzentration einer Lupus antikoagulans-auslösenden Substanz enthalten ist.

Bei den Gefäßen kann es sich um Glas- oder Kunststoffröhrchen oder Fläschchen handeln, die das Kalibratormaterial in flüssiger oder in lyophilisierter Form enthalten. In dem Fall, in dem lyophilisierte Kalibratormaterialien vorgesehen sind, enthält das Kalibratorset vorzugsweise ein weiteres Gefäß mit einem Lösungsmedium, wie beispielsweise eine Pufferlösung oder Wasser.

Sowohl die erfindungsgemäßen Kalibratormaterialien als auch die erfindungsgemäßen Kalibratorsets eignen sich zur Verwendung für die Kalibration von Gerinnungstesten, die für die Bestimmung von Lupus antikoagulans durchgeführt werden. Bekannte Gerinnungsteste, die für die Bestimmung von Lupus antikoagulans durchgeführt werden, sind beispielsweise die APTT, dAPTT, PT, dPT, KCT, dTT, Taipan Snake Venom Time und DRVVT.

Ein weiterer Gegenstand der vorliegenden Erfindung ist ein Verfahren zur Kalibration von Gerinnungstesten, die für die Bestimmung von Lupus antikoagulans durchgeführt werden. Das Verfahren umfasst die Schritte:
a) Durchführen eines ersten, Lupus antikoagulans-sensitiven Gerinnungstestes, wobei die Gerinnungszeit eines humanen Normalplasmas und die Gerinnungszeit mindestens eines erfindungsgemäßen Kalibratormaterials bestimmt wird;
b) Durchführen eines zweiten, Lupus antikoagulans-insensitiven Gerinnungstestes, wobei die Gerinnungszeit desselben humanen Normalplasmas und die Gerinnungszeit desselben Kalibratormaterials bestimmt wird;
c) Erstellen einer ersten Kalibrationskurve für den ersten, Lupus antikoagulans-sensitiven Gerinnungstest und einer zweiten Kalibrationskurve für den zweiten, Lupus antikoagulans-insensitiven Gerinnungstest, wobei den in den Schritten a) und b) bestimmten Gerinnungszeiten Werte in einer artifiziellen Einheit zugeordnet werden.

Bei der "artifiziellen Einheit" kann es sich um relative Zahlenwerte handeln, die die Gerinnungszeit-verlängernden Eigenschaften eines Kalibratormaterials größenordnungsmäßig angeben, z.B. der Wert 0 für einen Kalibrator mit vollständigem Gerinnungsfaktormangel (z.B. 0 % Faktor X) und gleichzeitig sehr hoher Konzentration an Lupus antikoagulans-auslösender Substanz und damit mit maximal Gerinnungszeit-verlängernden Eigenschaften; der Wert 100 für Normalplasma (mit 100 % Faktor X und ohne Lupus antikoagulans-auslösende Substanz) ohne Gerinnungszeit-verlängernde Eigenschaften ; und Werte zwischen 0 und 100 für Kalibratoren, die verglichen mit dem Kalibrator 0 zunehmende Gerinnungsfaktorkonzentrationen und abnehmende Konzentrationen an Lupus antikoagulans-auslösender Substanz aufweisen.

Vorzugsweise werden die Gerinnungszeiten von mindestens zwei erfindungsgemäßen Kalibratormaterialien bestimmt, wobei das erste Kalibratormaterial einen höheren Mangel an einem Gerinnungsfaktor und eine höhere Konzentration der Lupus antikoagulans-auslösenden Substanz aufweist als das zweite Kalibratormaterial und wobei dem ersten Kalibratormaterial ein geringerer Wert in einer artifiziellen Einheit zugeordnet ist als dem zweiten Kalibratormaterial.

Ein weiterer Gegenstand der vorliegenden Erfindung ist ein Verfahren zur Bestimmung von Lupus antikoagulans in einer Plasmaprobe eines Patienten. Das Verfahren umfasst die Schritte:
a) Durchführen eines ersten, Lupus antikoagulans-sensitiven Gerinnungstestes, wobei die Gerinnungszeit des Patientenplasmas bestimmt wird;
b) Durchführen eines zweiten, Lupus antikoagulans-insensitiven Gerinnungstestes, wobei die Gerinnungszeit desselben Patientenplasmas bestimmt wird;
c) Ermitteln des der jeweiligen bestimmten Gerinnungszeit zugeordneten Wertes in der artifiziellen Einheit an einer entsprechenden Kalibrationskurve, die mit einem erfindungsgemäßen Verfahren erstellt wurde;
d) Bildung des Quotienten aus den für das Patientenplasma bestimmten Werten; und
e) Feststellen von Lupus antikoagulans, wenn der gebildete Quotient einen vorbestimmten Referenzquotienten überschreitet.

Die Quotientenbildung (Ratiobildung) aus den beiden Gerinnungstest-Ergebnissen ist ein übliches Vorgehen bei der Diagnose von Lupus antikoagulans. Ein Referenzquotient wird bestimmt, indem eine statistisch hinreichende Anzahl von bekanntermaßen Lupus antikoagulans-positiven und von Lupus antikoagulans-negativen Patientenproben mit dem ersten und zweiten Gerinnungstest untersucht werden, die Quotienten gebildet werden und ein Quotientenwert (Grenzwert, Cut off-Wert) ermittelt wird, der eine möglichst sichere Unterscheidung zwischen Lupus antikoagulans-positiven und von Lupus antikoagulans-negativen Patientenproben ermöglicht.

Ein weiterer Gegenstand der vorliegenden Erfindung ist ein anderes Verfahren zur Kalibration von Gerinnungstesten, die für die Bestimmung von Lupus antikoagulans durchgeführt werden. Das Verfahren umfasst die Schritte:
a) Durchführen eines ersten, Lupus antikoagulans-sensitiven Gerinnungstestes, wobei die Gerinnungszeit eines humanen Normalplasmas und die Gerinnungszeit mindestens eines erfindungsgemäßen Kalibratormaterials bestimmt wird;
b) Durchführen eines zweiten, Lupus antikoagulans-insensitiven Gerinnungstestes, wobei die Gerinnungszeit desselben humanen Normalplasmas und die Gerinnungszeit desselben Kalibratormaterials bestimmt wird;
c) Bildung des Quotienten aus den Gerinnungszeiten des Normalplasmas und Bildung des Quotienten aus den Gerinnungszeiten des Kalibratormaterials;
d) Erstellen einer Kalibrationskurve, wobei den in Schritt c) bestimmten Quotienten Werte in einer artifiziellen Einheit zugeordnet werden.

Noch ein weiterer Gegenstand der vorliegenden Erfindung ist ein weiteres Verfahren zur Bestimmung von Lupus antikoagulans in einer Plasmaprobe eines Patienten. Das Verfahren umfasst die Schritte:
a) Durchführen eines ersten, Lupus antikoagulans-sensitiven Gerinnungstestes, wobei die Gerinnungszeit des Patientenplasmas bestimmt wird;
b) Durchführen eines zweiten, Lupus antikoagulans-insensitiven Gerinnungstestes, wobei die Gerinnungszeit desselben Patientenplasmas bestimmt wird;
c) Bildung des Quotienten aus den für das Patientenplasma bestimmten Gerinnungszeiten;
d) Ermitteln des dem bestimmten Quotienten zugeordneten Wertes in der artifiziellen Einheit an einer entsprechenden Kalibrationskurve, die mit dem oben beschriebenen Kalibrationsverfahren erstellt wurde; und
e) Feststellen von Lupus antikoagulans, wenn der ermittelte Wert einen vorbestimmten Referenzwert überschreitet.

Die Referenzwertbestimmung erfolgt in Analogie zu der oben beschriebenen Bestimmung eines Referenzquotienten.

In den beschriebenen Kalibrationsverfahren und Verfahren zur Bestimmung von Lupus antikoagulans unterscheiden sich der erste, Lupus antikoagulans-sensitive Gerinnungstest und der zweite, Lupus antikoagulans-insensitive Gerinnungstest typischerweise nur dadurch, dass in dem zweiten, Lupus antikoagulans-insensitiven Gerinnungstest mehr Phospholipide eingesetzt werden als in dem ersten, Lupus antikoagulans-sensitiven Gerinnungstest.

Dafür bekanntermaßen geeignete Gerinnungsteste sind ausgewählt aus der Gruppe APTT, dAPTT, PT, dPT, KCT, dTT, Taipan Snake Venom Time und DRVVT.

### FIGURENBESCHREIBUNG

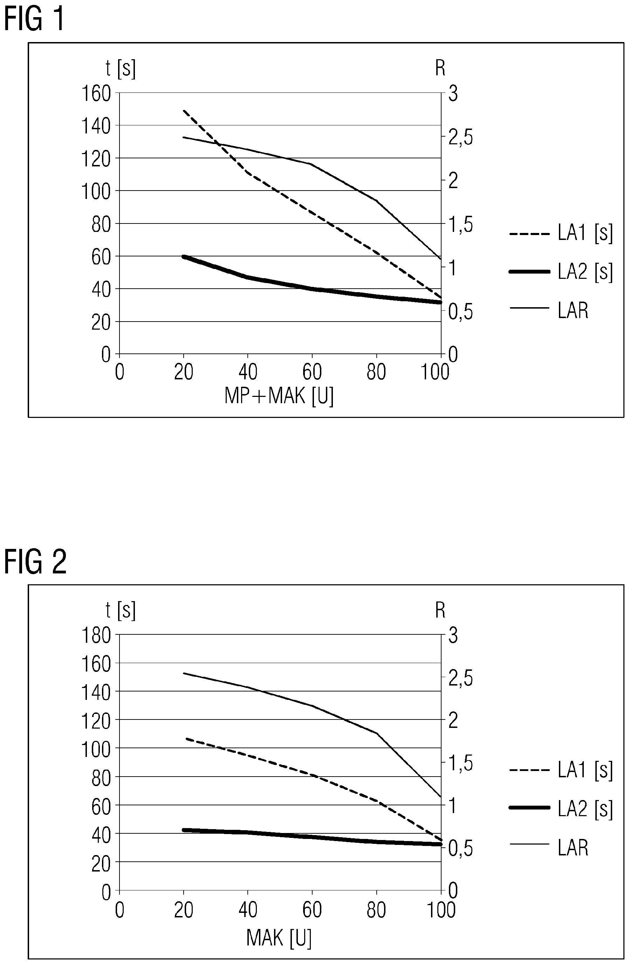

- FIG. 1: zeigt die erfindungsgemäß auf einem BCS-Gerinnungsanalyzer erstellten Kalibrationskurven für den Lupus antikoagulans-sensitiven DRVVT-Test (LA1), den Lupus antikoagulans-insensitiven DRVVT-Test (LA2) sowie für das Ratio aus den LA1- und LA2-Testergebnissen (LAR) ("MP+MAK-Kalibration"); die gemessenen LA1- und LA2-Gerinnungszeiten [s] bzw. das Ratio (R) wurden gegen artifizielle Einheiten [U] der Kalibratormaterialien aufgetragen, wobei der Wert 100 U für ein Normalplasma und der Wert 0 U für einen Kalibrator mit 0 % Faktor X und gleichzeitig sehr hoher Konzentration an Lupus antikoagulans-MAK steht.
- FIG. 2: zeigt auf einem BCS-Gerinnungsanalyzer erstellte Kalibrationskurven für den Lupus antikoagulanssensitiven DRVVT-Test (LA1), den Lupus antikoagulans-insensitiven DRVVT-Test (LA2) sowie für das Ratio aus den LA1- und LA2-Testergebnissen (LAR), die mit Kalibratoren, die im Unterschied zu den erfindungsgemäßen Kalibratoren keinen Gerinnungsfaktormangel aufwiesen, sondern nur den Lupus antikoagulans-MAK enthielten ("MAK-Kalibration"), erstellt wurden; die gemessenen LA1-und LA2-Gerinnungszeiten [s] bzw. das Ratio (R) wurden gegen die artifiziellen Einheiten [U] der Kalibratormaterialien aufgetragen, wobei der Wert 100 U für ein Normalplasma und der Wert 0 U für einen Kalibrator mit normalem Faktorengehalt und gleichzeitig sehr hoher Konzentration an Lupus antikoagulans-MAK steht.

Die folgenden Beispiele dienen der Veranschaulichung und sind nicht als Einschränkung zu verstehen.

Im Folgenden wird der Begriff "Lupus antikoagulans" mit "LA" abgekürzt.

### BEISPIELE

### BEISPIEL 1: Herstellung erfindungsgemäßer Kalibratoren

Zur Herstellung des Stammkalibratormaterials wurde einem humanem Faktor X Mangelplasma (0 % Faktor X; Siemens Healthcare Diagnostics Products GmbH) ein monoklonaler, Lupus antikoagulans-simulierender anti-Prothrombin Antikörper (Antikörper 195/097 hergestellt gemäß WO-A1-00/02925) zugegeben. Zu 1000 µL Mangelplasma wurden 5 µL einer Antikörperlösung (16,49 mg/mL) gegeben, so dass der Stammkalibrator (per definitionem 0 artifizielle Einheiten) eine Antikörperkonzentration von 82,0 µg/mL aufweist und keinen Faktor X enthält.

Zur Erstellung von Kalibratoren mit zunehmender Faktor X-Konzentration und abnehmender Antikörperkonzentration wurden Mischungen des Stammkalibrators mit humanem Normalplasma (Standard Human Plasma; Siemens Healthcare Diagnostics Products GmbH) in verschiedenen Mischungsverhältnissen hergestellt.

### BEISPIEL 2: Erstellung von Kalibrationskurven für einen LAsensitiven (LA1) und einen LA-insensitiven (LA2) DRVVT-Gerinnungstest

Mit den in Beispiel 1 beschriebenen Kalibratormaterialien wurden jeweils ein LA-sensitiver (LA1) und ein LAinsensitiver (LA2) DRVVT-Gerinnungstest mit dem LA1 Screeningreagenz beziehungsweise mit dem LA2 Bestätigungsreagenz (Siemens Healthcare Diagnostics Products GmbH) durchgeführt. Die Teste wurden automatisch an den Gerinnungsgeräten BCS und BCT (Siemens Healthcare Diagnostics Products GmbH) abgearbeitet. Am BCS-Gerät wurden 100 µL Probe und 100 µL des LA1 Screeningreagenzes beziehungsweise des LA2 Bestätigungsreagenzes (Siemens Healthcare Diagnostics Products GmbH) in unterschiedliche Küvetten des Rotors pipettiert und dann 230 Sekunden zwecks Temperierung inkubiert. Danach erfolgte die Mischung von Probe und Reagenz und die Reaktion startete. Das Eintreten der Gerinnung wurde photometrisch als Überschreitung einer Trübungsschwelle erfasst und als Gerinnungszeit [s] vom Gerät ausgegeben. Am BCT-Gerät wurden 100 µL Probe in die Messküvette gegeben, für 60 Sekunden inkubiert und dann mit 100 µL des LA1- oder des LA2-Reagenzes versetzt. Die Gerinnungszeit [s] wurde ebenfalls photometrisch erfasst. Zur Erstellung einer Kalibrationskurve wurden die Testergebnisse in Abhängigkeit der artifiziellen Enheiten der Kalibratormaterialien aufgetragen (vergleiche FIG. 1) und in der Gerätesoftware abgespeichert.

### BEISPIEL 3: Geräte- und Reagenzchargen-spezifische Kalibration von DRVVT-Messergebnissen LA-positiver und LAnegativer Proben

### BEISPIEL 3a): LA1- und LA2-Messwert-Kalibration an unterschiedlichen Geräten

Zur Untersuchung des Messwert-angleichenden Effektes einer erfindungsgemäßen Kalibration (mit den in Beispiel 1 beschriebenen Kalibratoren, "MP+MAK-Kalibration") eines DRVVT-basierten LA-Testes wurden verschiedene LA-positive und LA-negative Proben auf zwei verschiedenen automatischen Analysegeräten (BCS und BCT Analyzer, Siemens Healthcare Diagnostics Products GmbH) gemessen. Zum Vergleich wurde eine Kalibration mit Kalibratoren durchgeführt, die im Unterschied zu den erfindungsgemäßen Kalibratoren keinen Gerinnungsfaktormangel aufwiesen (also ungefähr 100 % Faktor X), sondern nur den monoklonalen, Lupus antikoagulanssimulierenden anti-Prothrombin Antikörper (Antikörper 195/097 in humanem Normalplasma (Standard Human Plasma; Siemens Healthcare Diagnostics Products GmbH) enthielten ("MAK-Kalibration"). Zur Erstellung einer Kalibrationskurve wurden die Testergebnisse in Abhängigkeit der artifiziellen Einheiten der Kalibratormaterialien aufgetragen (vergleiche FIG. 2) und in der Gerätesoftware abgespeichert.

In Tabelle 1 sind die relativen Differenzen (%) zwischen den auf den beiden Geräten gemessenen Rohwerten (LA1-Gerinnungszeit in Sekunden) und zwischen den kalibrierten LA1-Werten (artifizielle Einheiten [U]) für die verschiedenen Probentypen:
- Standardhumanplasma (SHP),
- Plasmakontrolle mit relativ niedriger LA-Aktivität (LA Kontrolle niedrig),
- Plasmakontrolle mit relativ hoher LA-Aktivität (LA Kontrolle hoch),
- ein Plasma bestehend aus einer Mischung von 80 % SHP und 20 % Faktor X-Mangelplasma mit 0 % Faktor X (80 % SHP+ 20 % FX-MP),
- Normalplasma und
- eine LA-positive Patientenprobe (LA-Probe),
gezeigt.

Anhand des Mittelwerts der relativen Differenzen zwischen BCS- und BCT-Testergebnissen wird deutlich, dass die Abweichungen der LA1-Werte zwischen den beiden Geräten am geringsten sind, wenn eine erfindungsgemäße Kalibration (MP+MAK-Kalibration) durchgeführt wird.

**Tabelle 1: Relative Differenzen der LA1-Gerinnungszeiten vor und nach Kalibration auf zwei unterschiedlichen Analysegeräten**

| | | **Differenz BCT vs. BCS [%]** | | |
|---|---|---|---|---|
| | **Probe Nr.** | **Rohwerte** | **MAK-Kalibration** | **MP+MAK-Kalibration** |
| SHP | 1 | 8,0 | 0,1 | 0,9 |
| LA Kontrolle niedrig | 2 | 13,3 | -4,5 | -3,2 |
| LA Kontrolle hoch | 3 | 14,3 | -21,5 | -10,3 |
| 80% SHP+ 20 % FX-MP | 4 | 6, 8 | 0,3 | 1,1 |
| Normalplasma | 5 | 2,5 | 0,7 | 1,8 |
| LA-Probe | 6 | -2,7 | 4,0 | 6,1 |
| **Mittelwert** | - | **7,0** | **-3,5** | **-0,6** |

In Tabelle 2 sind die relativen Differenzen (%) zwischen den auf den beiden Geräten gemessenen Rohwerten (LA2-Gerinnungszeit in Sekunden) und zwischen den kalibrierten LA2-Gerinnungszeiten (artifizielle Einheiten [U]) für die gleichen Probentypen wie in Tabelle 1 gezeigt.

Anhand des Mittelwerts der relativen Differenzen zwischen BCS- und BCT-Messwerten wird deutlich, dass die Abweichungen der LA2-Messwerte zwischen den beiden Geräten am geringsten sind, wenn eine erfindungsgemäße Kalibration (MP+MAK-Kalibration) durchgeführt wird. Zwei Rohwerte (für Probe Nr. 5 und 6) konnten mit der MAK-Kalibration nicht kalibriert werden, weil die Kalibrationskurve in diesem Bereich nur eine sehr geringe Spreizung aufwies.

**Tabelle 2: Relative Differenzen der LA2-Gerinnungszeiten vor und nach Kalibration auf zwei unterschiedlichen Analysegeräten**

| | | **Differenz BCT vs. BCS [%]** | | |
|---|---|---|---|---|
| | **Probe Nr.** | **Rohwerte** | **MAK-Kalibration** | **MP+MAK-Kalibration** |
| SHP | 1 | 14,7 | -3,9 | 0,0 |
| LA Kontrolle niedrig | 2 | 14,5 | -9,4 | -2,3 |
| LA Kontrolle hoch | 3 | 14,0 | -7,8 | -1,6 |
| 80% SHP+ 20 % FX-MP | 4 | 13,4 | -3,1 | 0,5 |
| Normalplasma | 5 | 9,7 | n.a. | 4,0 |
| LA-Probe | 6 | 4,7 | n.a. | 29,7 |
| **Mittelwert** | - | **11,8** | **n.a.** | **5,1** |

### BEISPIEL 3b): LA1- und LA2-Messwert-Kalibration mit unterschiedlichen Reagenzchargen

Zur Untersuchung des Messwert-angleichenden Effektes einer erfindungsgemäßen Kalibration (mit den in Beispiel 1 beschriebenen Kalibratoren, "MP+MAK-Kalibration") eines DRVVT-basierten LA-Testes wurden verschiedene LA-positive und LA-negative Proben mit verschiedenen Chargen der DRVVT-Reagenzien gemessen.

In Tabelle 3 sind die relativen Differenzen (%) zwischen den mit den verschiedenen Reagenzchargen (Chargen 1-4) gemessenen Rohwerten (LA1-Gerinnungszeit in Sekunden) und zwischen den kalibrierten LA1-Werten (artifizielle Einheiten) für die verschiedenen Probentypen:
- Standardhumanplasma (SHP),
- Plasmakontrolle mit relativ niedriger LA-Aktivität (LA Kontrolle niedrig),
- Plasmakontrolle mit relativ hoher LA-Aktivität (LA Kontrolle hoch),
- ein Plasma bestehend aus einer Mischung von 20 % SHP und 80 % Faktor X-Mangelplasma mit 0 % Faktor X (20 % SHP+ 80 % FX-MP), und
- ein Plasma bestehend aus einer Mischung von 20 % SHP und 80 % Faktor II-Mangelplasma mit 0 % Faktor II (20 % SHP+ 80 % FII-MP),
gezeigt.

Anhand des Mittelwerts der relativen Differenzen zwischen Messwerten der Charge 1 und den Messwerten der Chargen 2, 3 und 4 wird deutlich, dass die Abweichungen der LA1-Messwerte zwischen den verschiedenen Chargen vermindert werden, wenn eine erfindungsgemäße Kalibration (MP+MAK-Kalibration) durchgeführt wird.

**Tabelle 3: Relative Differenzen der LA1-Gerinnungszeiten vor und nach Kalibration zwischen unterschiedlichen DRVVT-Reagenzchargen**

| | | **Differenz Charge 1 vs. Chargen 2-4 [%]** | | | | | |
|---|---|---|---|---|---|---|---|
| | | **Rohwerte** | | | **MP+MAK-Kalibration** | | |
| | **Probe Nr.** | **1-2** | **1-3** | **1-4** | **1-2** | **1-3** | **1-4** |
| SHP | 1 | -15,9 | -13,0 | -16,7 | -0,5 | -0,5 | 0,0 |
| LA Kontrolle niedrig | 2 | 2,5 | 6,0 | 1,4 | -3,8 | -4,3 | -2,7 |
| LA Kontrolle hoch | 3 | 11,0 | 14,5 | 10,9 | -8,6 | -9,5 | -7,8 |
| 20 % SHP+ 80 % FX-MP | 4 | -15,5 | -10,7 | -16,1 | 4,1 | 3,4 | 4,9 |
| 20 % SHP+ 80 % FII-MP | 5 | -25,4 | -21,9 | -27,6 | 8,9 | 8,9 | 10,5 |
| **Mittelwert** | - | **-7,8** | | | **0,2** | | |

In Tabelle 4 sind die relativen Differenzen (%) zwischen den mit den verschiedenen Reagenzchargen (Chargen 1-4) gemessenen Rohwerten (LA2-Gerinnungszeit in Sekunden) und zwischen den kalibrierten LA2-Werten (artifizielle Einheiten) für die gleichen Probentypen wie in Tabelle 3 gezeigt.

Anhand des Mittelwerts der relativen Differenzen zwischen Messwerten der Charge 1 und den Messwerten der Chargen 2, 3 und 4 wird deutlich, dass die Abweichungen der LA2-Messwerte zwischen den verschiedenen Chargen vermindert werden, wenn eine erfindungsgemäße Kalibration (MP+MAK-Kalibration) durchgeführt wird.

**Tabelle 4: Relative Differenzen der LA2-Gerinnungszeiten vor und nach Kalibration zwischen unterschiedlichen DRVVT-Reagenzchargen**

| | | **Differenz Charge 1 vs. Chargen 2-4 [%]** | | | | | |
|---|---|---|---|---|---|---|---|
| | | **Rohwerte** | | | **MP+MAK-Kalibration** | | |
| | **Probe Nr.** | **1-2** | **1-3** | **1-4** | **1-2** | **1-3** | **1-4** |
| SHP | 1 | 11,0 | 5,8 | 5,2 | -2,2 | -1,8 | 1,0 |
| LA Kontrolle niedrig | 2 | 11,1 | 6,4 | 4,7 | -5,5 | -6,9 | 1,8 |
| LA Kontrolle hoch | 3 | 12,0 | 6, 6 | 5,4 | -8,5 | -8,5 | -0,8 |
| 20 % SHP+ 80 % FX-MP | 4 | 10,5 | 4,5 | 5,1 | -0,8 | -2,8 | -2,8 |
| 20 % SHP+ 80 % FII-MP | 5 | 10,1 | 4,4 | 3,3 | -0,6 | -1,3 | 2,5 |
| **Mittelwert** | - | **7,1** | | | **-2,5** | | |

### BEISPIEL 3c): LA1/LA2-Ratio-Kalibration an unterschiedlichen Geräten

Zur Untersuchung des Effektes der Angleichung der LA1/LA2-Ratio-Werte durch eine erfindungsgemäßen Kalibration wurden zwei Varianten der Ratio-Kalibration (mit den in Beispiel 1 beschriebenen Kalibratoren, "MP+MAK-Kalibration") für einen DRVVT-basierten LA-Test und für verschiedene LA-positive und LA-negative Proben auf zwei verschiedenen automatischen Analysegeräten (BCS und BCT Analyzer, Siemens Healthcare Diagnostics Products GmbH) durchgeführt.

Bei der ersten Variante wurden bereits erfindungsgemäß kalibrierte LA1- und LA2-Werte (gemäß Beispiel 3a) für die Berechnung des LA1/LA2-Ratios verwendet ("Variante 1").

Bei der zweiten Variante wurde das LA1/LA2-Ratio aus den unkalibrierten Rohwerten der LA1- und LA2-Gerinnungszeiten gebildet und dann an einer vorab erstellten Kalibrationskurve abgelesen, bei der LA1/LA2-Ratios von Kalibratoren bekannter Zusammensetzung (mit den in Beispiel 1 beschriebenen Kalibratoren, "MP+MAK-Kalibration") bestimmt worden waren.

In Tabelle 5 sind die relativen Differenzen (%) zwischen den Ratios der auf den beiden Geräten gemessenen Rohwerte und zwischen den kalibrierten Ratios gemäß Variante 1 und Variante 2 für die gleichen Probentypen wie in Tabelle 1 gezeigt.

Betrachtet man die einzelnen relativen Differenzen zwischen den BCS- und BCT-Ratios wird deutlich, dass die Abweichungen der LA1/LA2-Ratios zwischen den beiden Geräten für die meisten Proben geringer wird, wenn eine erfindungsgemäße Kalibration (MP+MAK-Kalibration) durchgeführt wird. Die Mittelwerte sind hier weniger aussagekräftig, da in beiden Kalibrationsvarianten jeweils eine Probe ein stark abweichendes Ergebnis lieferte.

**Tabelle 5: Relative Differenzen der LA1/LA2-Ratios vor und nach Kalibration auf zwei unterschiedlichen Analysegeräten**

| | | **Differenz BCS vs. BCT [%]** | | |
|---|---|---|---|---|
| | **Probe Nr.** | **Rohwerte** | **MP+MAK-Kalibration Variante 1** | **MP+MAK-Kalibration Variante 2** |
| SHP | 1 | -7,5 | 0,9 | 0,7 |
| LA Kontrolle niedrig | 2 | -1,4 | -0,9 | -3,0 |
| LA Kontrolle hoch | 3 | 0,3 | -8,5 | -24,9 |
| 80% SHP+ 20 % FX-MP | 4 | -7,7 | 0,6 | 1,1 |
| Normalplasma | 5 | -8,0 | -2,3 | 1,0 |
| LA-Probe | 6 | -7,8 | -33,7 | 0,5 |
| **Mittelwert** | - | **-5,4** | **-7,3** | **-4,1** |

## Patentansprüche

1. Kalibratormaterial bestehend aus Plasma, das im Vergleich zu humanem Normalplasma einen Mangel an mindestens einem Gerinnungsfaktor aus der Gruppe Faktor II und Faktor X aufweist, wobei das Plasma im Vergleich zu humanem Normalplasma 0 % Faktor II und/oder 0 % Faktor X enthält und ferner mindestens eine Lupus antikoagulans-auslösende Substanz enthält.

2. Kalibratormaterial gemäß Anspruch 1, wobei das Plasma als Lupus antikoagulans-auslösende Substanz mindestens einen Antiphospholipid-Antikörper enthält, vorzugsweise einen, zwei oder mehr Antiphospholipid-Antikörper aus der Gruppe anti-β2-Glykoprotein I-Antikörper, anti-Prothrombin-Antikörper, anti-Cardiolipin-Antikörper, anti-Protein C-Antikörper, anti-Protein S-Antikörper, anti-Thrombomodulin-Antikörper und anti-Faktor XII-Antikörper enthält.

3. Kalibratormaterial gemäß Anspruch 2, wobei der jeweilige Antikörper in einer Konzentration von 0,5 bis 500 µg/mL enthalten ist.

4. Kalibratormaterial gemäß einem der Ansprüche 1 und 2, das als Lupus antikoagulans-auslösende Substanz mindestens ein Annexin enthält, vorzugsweise Annexin V.

5. Kalibratormaterial gemäß Anspruch 4, wobei das jeweilige Annexin in einer Konzentration von 0,5 bis 500 µg/mL enthalten ist.

6. Verfahren zur Herstellung eines Kalibratormaterials gemäß einem der Ansprüche 1 bis 5, wobei Plasma, das im Vergleich zu humanem Normalplasma 0 % Faktor II und/oder 0 % Faktor X enthält, eine definierte Menge mindestens einer Lupus antikoagulans-auslösenden Substanz zugegeben wird.

7. Kalibratorset enthaltend ein erstes Gefäß enthaltend ein Kalibratormaterial gemäß einem der Ansprüche 1 bis 5 und optional ein zweites Gefäß enthaltend humanes Normalplasma.

8. Verwendung mindestens eines Kalibratormaterials gemäß einem der Ansprüche 1 bis 5 oder eines Kalibratorsets gemäß Anspruch 7 zur Kalibration von Gerinnungstesten, die für die Bestimmung von Lupus antikoagulans durchgeführt werden.

9. Verfahren zur Kalibration von Gerinnungstesten, die für die Bestimmung von Lupus antikoagulans durchgeführt werden, das Verfahren umfassend die Schritte:
a) Durchführen eines ersten, Lupus antikoagulans-sensitiven Gerinnungstestes, wobei die Gerinnungszeit eines humanen Normalplasmas und die Gerinnungszeit mindestens eines Kalibratormaterials gemäß einem der Ansprüche 1 bis 5 bestimmt wird;
b) Durchführen eines zweiten, Lupus antikoagulans-insensitiven Gerinnungstestes, wobei die Gerinnungszeit desselben humanen Normalplasmas und die Gerinnungszeit desselben Kalibratormaterials bestimmt wird;
c) Erstellen einer ersten Kalibrationskurve für den ersten, Lupus antikoagulans-sensitiven Gerinnungstest und einer zweiten Kalibrationskurve für den zweiten, Lupus antikoagulans-insensitiven Gerinnungstest, wobei den in den Schritten a) und b) bestimmten Gerinnungszeiten Werte in einer artifiziellen Einheit zugeordnet werden.

10. Verfahren gemäß Anspruch 9, wobei die Gerinnungszeiten von mindestens zwei Kalibratormaterialien bestimmt werden, wobei das erste Kalibratormaterial einem der Ansprüche 1-5 entspricht und damit einen höheren Mangel an einem Gerinnungsfaktor und eine höhere Konzentration der Lupus antikoagulans-auslösenden Substanz aufweist als das zweite Kalibratormaterial, welches durch Zugabe von humanem Normalplasma zu einem Kalibratormaterial gemäß einem der Ansprüche 1-5 herstellbar ist, und wobei dem ersten Kalibratormaterial ein geringerer Wert in der artifiziellen Einheit zugeordnet ist als dem zweiten Kalibratormaterial.

11. Verfahren zur Bestimmung von Lupus antikoagulans in einer Plasmaprobe eines Patienten, das Verfahren umfassend die Schritte:
a) Durchführen eines ersten, Lupus antikoagulans-sensitiven Gerinnungstestes, wobei die Gerinnungszeit des Patientenplasmas bestimmt wird;
b) Durchführen eines zweiten, Lupus antikoagulans-insensitiven Gerinnungstestes, wobei die Gerinnungszeit desselben Patientenplasmas bestimmt wird;
c) Ermitteln des der jeweiligen bestimmten Gerinnungszeit zugeordneten Wertes in der artifiziellen Einheit an einer entsprechenden Kalibrationskurve, die mit einem Verfahren gemäß Anspruch 9 erstellt wurde;
d) Bildung des Quotienten aus den für das Patientenplasma bestimmten Werten; und
e) Feststellen von Lupus antikoagulans, wenn der gebildete Quotient einen vorbestimmten Referenzquotienten überschreitet.

12. Verfahren zur Kalibration von Gerinnungstesten, die für die Bestimmung von Lupus antikoagulans durchgeführt werden, das Verfahren umfassend die Schritte:
a) Durchführen eines ersten, Lupus antikoagulans-sensitiven Gerinnungstestes, wobei die Gerinnungszeit eines humanen Normalplasmas und die Gerinnungszeit mindestens eines Kalibratormaterials gemäß einem der Ansprüche 1 bis 5 bestimmt wird;
b) Durchführen eines zweiten, Lupus antikoagulans-insensitiven Gerinnungstestes, wobei die Gerinnungszeit desselben humanen Normalplasmas und die Gerinnungszeit desselben Kalibratormaterials bestimmt wird;
c) Bildung des Quotienten aus den Gerinnungszeiten des Normalplasmas und Bildung des Quotienten aus den Gerinnungszeiten des Kalibratormaterials;
d) Erstellen einer Kalibrationskurve, wobei den in Schritt c) bestimmten Quotienten Werte in einer artifiziellen Einheit zugeordnet werden.

13. Verfahren zur Bestimmung von Lupus antikoagulans in einer Plasmaprobe eines Patienten, das Verfahren umfassend die Schritte:
a) Durchführen eines ersten, Lupus antikoagulans-sensitiven Gerinnungstestes, wobei die Gerinnungszeit des Patientenplasmas bestimmt wird;
b) Durchführen eines zweiten, Lupus antikoagulans-insensitiven Gerinnungstestes, wobei die Gerinnungszeit desselben Patientenplasmas bestimmt wird;
c) Bildung des Quotienten aus den für das Patientenplasma bestimmten Gerinnungszeiten;
d) Ermitteln des dem bestimmten Quotienten zugeordneten Wertes in der artifiziellen Einheit an einer entsprechenden Kalibrationskurve, die mit einem Verfahren gemäß Anspruch 12 erstellt wurde; und
e) Feststellen von Lupus antikoagulans, wenn der ermittelte Wert einen vorbestimmten Referenzwert überschreitet.

14. Verfahren gemäß einem der Ansprüche 9 bis 13, wobei der erste, Lupus antikoagulans-sensitive Gerinnungstest und der zweite, Lupus antikoagulans-insensitive Gerinnungstest sich nur dadurch unterscheiden, dass in dem zweiten, Lupus antikoagulans-insensitiven Gerinnungstest mehr Phospholipide eingesetzt werden als in dem ersten, Lupus antikoagulans-sensitiven Gerinnungstest.

15. Verfahren gemäß Anspruch 14, wobei die Gerinnungsteste ausgewählt sind aus der Gruppe APTT, dAPTT, PT, dPT, KCT, dTT, Taipan Snake Venom Time und DRVVT.

## Claims

1. Calibrator material consisting of plasma which, in comparison with human normal plasma, has a deficiency of at least one coagulation factor from the group consisting of factor II and factor X, wherein the plasma contains 0% factor II and/or 0% factor X in comparison with human normal plasma and further contains at least one lupus anticoagulant-causing substance.

2. Calibrator material according to Claim 1, wherein the plasma contains, as lupus anticoagulant-causing substance, at least one anti-phospholipid antibody, preferably contains one, two or more anti-phospholipid antibodies from the group consisting of anti-β2-glycoprotein I antibody, anti-prothrombin antibody, anti-cardiolipin antibody, anti-protein C antibody, anti-protein S antibody, anti-thrombomodulin antibody and anti-factor XII antibody.

3. Calibrator material according to Claim 2, wherein the antibody in question is present in a concentration of 0.5 to 500 µg/ml.

4. Calibrator material according to either of Claims 1 and 2, which contains at least one annexin, preferably annexin V, as lupus anticoagulant-causing substance.

5. Calibrator material according to Claim 4, wherein the annexin in question is present in a concentration of 0.5 to 500 µg/ml.

6. Method for producing a calibrator material according to any of Claims 1 to 5, wherein a defined amount of at least one lupus anticoagulant-causing substance is added to plasma containing 0% factor II and/or 0% factor X in comparison with human normal plasma.

7. Calibrator set containing a first vessel containing a calibrator material according to any of Claims 1 to 5 and optionally a second vessel containing human normal plasma.

8. Use of at least one calibrator material according to any of Claims 1 to 5 or of a calibrator set according to Claim 7 for calibration of coagulation tests which are carried out for the determination of lupus anticoagulant.

9. Method for calibrating coagulation tests which are carried out for the determination of lupus anticoagulant, the method comprising the steps of:
a) carrying out a first, lupus anticoagulant-sensitive coagulation test, wherein the coagulation time of a human normal plasma and the coagulation time of at least one calibrator material according to any of Claims 1 to 5 is determined;
b) carrying out a second, lupus anticoagulant-insensitive coagulation test, wherein the coagulation time of the same human normal plasma and the coagulation time of the same calibrator material is determined;
c) creating a first calibration curve for the first, lupus anticoagulant-sensitive coagulation test and a second calibration curve for the second, lupus anticoagulant-insensitive coagulation test, wherein values in an artificial unit are assigned to the coagulation times determined in steps a) and b) .

10. Method according to Claim 9, wherein the coagulation times of at least two calibrator materials are determined, wherein the first calibrator material corresponds to any of Claims 1-5 and hence has a higher deficiency of a coagulation factor and a higher concentration of the lupus anticoagulant-causing substance than the second calibrator material which is producible by addition of human normal plasma to a calibrator material according to any of Claims 1-5, and wherein a lower value in the artificial unit is assigned to the first calibrator material than to the second calibrator material.

11. Method for determining lupus anticoagulant in a plasma sample of a patient, the method comprising the steps of:
a) carrying out a first, lupus anticoagulant-sensitive coagulation test, wherein the coagulation time of the patient plasma is determined;
b) carrying out a second, lupus anticoagulant-insensitive coagulation test, wherein the coagulation time of the same patient plasma is determined;
c) ascertaining the value in the artificial unit assigned to the respective determined coagulation time, on a corresponding calibration curve which was created using a method according to Claim 9;
d) forming the quotient from the values determined for the patient plasma; and
e) establishing lupus anticoagulant if the quotient formed exceeds a predetermined reference quotient.

12. Method for calibrating coagulation tests which are carried out for the determination of lupus anticoagulant, the method comprising the steps of:
a) carrying out a first, lupus anticoagulant-sensitive coagulation test, wherein the coagulation time of a human normal plasma and the coagulation time of at least one calibrator material according to any of Claims 1 to 5 is determined;
b) carrying out a second, lupus anticoagulant-insensitive coagulation test, wherein the coagulation time of the same human normal plasma and the coagulation time of the same calibrator material is determined;
c) forming the quotient from the coagulation times of the normal plasma and forming the quotient from the coagulation times of the calibrator material;
d) creating a calibration curve, wherein values in an artificial unit are assigned to the quotients determined in step c).

13. Method for determining lupus anticoagulant in a plasma sample of a patient, the method comprising the steps of:
a) carrying out a first, lupus anticoagulant-sensitive coagulation test, wherein the coagulation time of the patient plasma is determined;
b) carrying out a second, lupus anticoagulant-insensitive coagulation test, wherein the coagulation time of the same patient plasma is determined;
c) forming the quotient from the coagulation times determined for the patient plasma;
d) ascertaining the value in the artificial unit assigned to the determined quotient, on a corresponding calibration curve which was created using a method according to Claim 12; and
e) establishing lupus anticoagulant if the value ascertained exceeds a predetermined reference value.

14. Method according to any of Claims 9 to 13, wherein the first, lupus anticoagulant-sensitive coagulation test and the second, lupus anticoagulant-insensitive coagulation test only differ in that more phospholipids are used in the second, lupus anticoagulant-insensitive coagulation test than in the first, lupus anticoagulant-sensitive coagulation test.

15. Method according to Claim 14, wherein the coagulation tests are selected from the group consisting of APTT, dAPTT, PT, dPT, KCT, dTT, Taipan snake venom time and DRVVT.

## Revendications

1. Matière d'étalonnage constituée de plasma, qui, par rapport au plasma humain normal, a un manque d'au moins un facteur de coagulation du groupe facteur II et facteur X, le plasma contenant, par rapport au plasma humain normal, 0% de facteur II et/ou 0% de facteur X et contenant, en outre, au moins une substance déclenchant un anticoagulant lupique.

2. Matière d'étalonnage suivant la revendication 1, dans laquelle le plasma contient, comme substance déclenchant un anticoagulant lupique, au moins un anticorps d'antiphospholipide, de préférence un, deux ou plusieurs anticorps d'antiphopholipide du groupe anticorps d'anti-β2-glycoprotéine I, anticorps d'anti-prothrombine, anticorps d'anti-cardiolipine, anticorps d'anti-protéine C, anticorps d'anti-protéine S, anticoprs d'anti-thrombomoduline et anticorps d'anti-facteur XII.

3. Matière d'étalonnage suivant la revendication 2, dans laquelle l'anticorps respectif est contenu en une concentration de 0,5 à 500 µg/mL.

4. Matière d'étalonnage suivant l'une des revendications 1 et 2, qui contient, comme substance déclenchant un anticoagulant lupique, au moins une annexine, de préférence l'annexine V.

5. Matière d'étalonnage suivant la revendication 4, dans laquelle l'annexine respective est contenue en une concentration de 0,5 à 500 µg/mL.

6. Procédé de préparation d'une matière d'étalonnage suivant l'une des revendications 1 à 5, dans lequel à du plasma, qui contient par rapport à du plasma humain normal, 0% de facteur II et/ou 0% de facteur X, on ajoute une quantité définie d'au moins une substance déclenchant un anticoagulant lupique.

7. Trousse d'étalonnage, contenant un premier récipient contenant une matière d'étalonnage suivant l'une des revendications 1 à 5 et, éventuellement, un deuxième récipient contenant du plasma humain normal.

8. Utilisation d'au moins une matière d'étalonnage suivant l'une des revendications 1 à 5, ou d'une trousse d'étalonnage suivant la revendication 7, pour l'étalonnement de tests de coagulation, qui sont effectués pour la détermination d'anticoagulants lupiques.

9. Procédé d'étalonnage de tests de coagulation, qui sont effectués pour la détermination d'anticoagulants lupique, le procédé comprenant les stades dans lesquels :
a) on effectue un premier test de coagulation sensible à un anticoagulant lupique, la durée de coagulation d'un plasma humain normal et la durée de coagulation d'au moins une matière d'étalonnage étant déterminées suivant l'une des revendications 1 à 5 ;
b) on effectue un deuxième test de coagulation insensible à un anticoagulant lupique, la durée de coagulation du même plasma humain normal et la durée de coagulation de la même matière d'étalonnage étant déterminées ;
c) on établit une première courbe d'étalonnage pour le premier test de coagulation sensible à un anticoagulant lupique et une deuxième courbe d'étalonnage pour le deuxième test de coagulation insensible à un anticoagulant lupique, dans lequel on associe, suivant une unité artificielle, des valeurs aux temps de coagulation déterminées au stade a) et b).

10. Procédé suivant la revendication 9, dans lequel on détermine les temps de coagulation d'au moins deux matières d'étalonnage, dans lequel la première matière d'étalonnage correspond à l'une des revendications 1 à 5 et a ainsi un manque plus grand de facteur de coagulation et une concentration plus grande de substance déclenchant un anticoagulant lupique que la deuxième matière d'étalonnage, qui peut être préparée par addition de plasma humain normal à une matière d'étalonnage suivant l'une des revendications 1 à 5, et dans lequel, à la première matière d'étalonnage est associée une valeur plus petite suivant l'unité artificielle qu'à la deuxième matière d'étalonnage.

11. Procédé de détermination d'anticoagulant lupique dans un échantillon de plasma d'un patient, le procédé comprenant les stades dans lesquels :
a) on effectue un premier test de coagulation sensible à un anticoagulant lupique, le temps de coagulation du plasma du patient étant déterminé ;
b) on effectue un deuxième test de coagulation insensible à un anticoagulant lupique, le temps de coagulation du même plasma du patient étant déterminé ;
c) on détermine la valeur, associée au temps de coagulation respectif déterminé, suivant l'unité artificielle sur une courbe d'étalonnage correspondante, qui a été établie par un procédé suivant la revendication 9 ;
d) on forme le quotient des valeurs déterminées pour le plasma du patient ; et
e) on constate de l'anticoagulant lupique, si le quotient formé dépasse un quotient de référence déterminé à l'avance.

12. Procédé d'étalonnage de tests de coagulation, que l'on effectue pour la détermination d'anticoagulant lupique, le procédé comprenant les stades dans lesquels :
a) on effectue un premier test de coagulation sensible à un anticoagulant lupique, le temps de coagulation d'un plasma humain normal et le temps de coagulation d'au moins une matière d'étalonnage, suivant l'une des revendications 1 à 5, étant déterminés ;
b) on effectue un deuxième test de coagulation insensible à un anticoagulant lupique, le temps de coagulation du même plasma humain normal et le temps de coagulation de la même matière d'étalonnage étant déterminés ;
c) on forme le quotient des temps de coagulation du plasma normal et on forme le quotient des temps de coagulation de la matière d'étalonnage ;
d) on établit une courbe d'étalonnage, dans lequel, au quotient déterminé au stade c), on associe des valeurs suivant une unité artificielle.

13. Procédé de détermination d'anticoagulant lupique dans un échantillon de plasma d'un patient, le procédé comprenant les stades dans lesquels :
a) on effectue un premier test de coagulation sensible à un anticoagulant lupique, le temps de coagulation du plasma du patient étant déterminé ;
b) on effectue un deuxième test de coagulation insensible à un anticoagulant lupique, le temps de coagulation du même plasma du patient étant déminé ;
c) on forme le quotient des temps de coagulation déterminés pour le plasma du patient ;
d) on détermine la valeur associée au quotient déterminé suivant l'unité artificielle sur une courbe d'étalonnage correspondante, qui a été établie par un procédé suivant la revendication 12 ; et
e) on constate de l'anticoagulant lupique, si la valeur déterminée dépasse une valeur de référence déterminée à l'avance.

14. Procédé suivant l'une des revendications 9 à 13, dans lequel le premier test de coagulation sensible à un anticoagulant lupique et le deuxième test de coagulation insensible à un anticoagulant lupique ne se distinguent qu'en ce que, dans le deuxième test insensible à un anticoagulant lupique, on utilise plus de phospholipide que dans le premier test de coagulation sensible à un anticoagulant lupique.

15. Procédé suivant la revendication 14, dans lequel les tests de coagulation sont choisis dans le groupe APTT, dAPTT, PT, dPT, KCT, dTT, Taipan Snake Venom Time et DRVVT.
